# EUROPEAN PATENT APPLICATION

(11) **EP 1 417 942 A2**
(43) Date of publication of application: **12.05.2004**
(21) Application number: 03256284.5
(22) Date of filing: 06.10.2003
(51) Int. Cl.: A61F 2/64, A61F 2/68

(54) **Prosthetic knee system**

(30) Priority: 29.10.2002 US 422025 P; 26.09.2003 US 670353
(71) Applicant: Molino, Joseph L., Valley Cottage, NY 10989 (US); Rebarber, Michael, Glen Rock, New Jersey 07452 (US)
(72) Inventor: Molino, Joseph L., Valley Cottage, NY 10989 (US); Rebarber, Michael, Glen Rock, New Jersey 07452 (US)
(74) Representative: Rees, Alexander Ellison

(57) **Abstract**

A prosthetic knee cage (10) consists of a base unit (12) formed by spaced from each other first and second side members (22,24) interconnected by a connecting element (26), so as to form an operational channel (28) therebetween. A platform (14) is adapted for pivotal cooperation with the base unit (12). An adjustment device (16) is provided for adjustment of pivotal movement of the platform (14) relative to the base unit (12).

## Description

### Background of the Invention

It is known that the stability of a person is a function of the posture of the person's body. The weight line is an imaginary line through which the center of gravity of the person's body projects into the ground. The stability of the knee is directly relational to the distance between the weight line and the knee axis. In the prosthetic knee devices stability is accomplished when the center of gravity and the weight line passing therethrough are placed anterior to the knee center, at the greatest possible distance therebetween. However, typically, the dynamics and the structure of prosthetic knee cages force the knee center to be maintained posterior relative to the weight line. The knee cages of the prior art, while trying to accomplish stability, typically provide a relatively minor degree of adjustments of the center of gravity and weight line of a patient relative to the pivotal axis of the cage. Significantly, the knee cage of the invention provides the ability to manipulate and position the weight line anterior to the knee center. To assure stability the knee cage of the invention utilizes a simple system that allows the knee unit to effectively shift the weight line up to four inches anterior or posterior relative to the center of rotation of the knee axis.

The terminal impact is known to be the resultant force generated during the swinging motion of a lower limb and an upper limb with respect to each other, when the prosthetic leg reaches the end of its swing phase, prior to achieving a substantially straight position. When humans advance in the walking cycle, a leg bends at the knee and is lifted above the ground. In order to be supported by the ground again, the leg has to be fully extended. Prosthetic legs operate in a similar manner. If the motion of the prosthetic lower limb caused by the forces of the terminal impact is not decelerated, a very revealing and often embarrassing noise is produced. To the discomfort of a patient, this makes the presence of the prosthesis very obvious, causing other people to be aware that the patient has an artificial leg. The terminal impact also negatively affects the structural elements of the prosthetic device itself, often leading to their damage. Thus, there is strong necessity of minimizing the effect of the terminal impact.

Another negative aspect of the prior art prosthetic devices is the artificially high rate at which the leg advances during the swing phase to its complete straight position. In the prosthetic devices, in the process of reaching the stop in full extension, an undesirable momentum of forces develops which has to be minimized. If this does not occur, one part of the leg will crash against the other part of the leg. Therefore, there is an obvious need for a prosthetic device capable of regulating and decelerating its motion during the swing phase before it reaches a substantially straight position. The prosthetic device of the invention closely simulates the movement of human limbs, without being subjected to the results of the terminal impact. It moves rapidly in the initial stages of extension, and then slows down before reaching the stop in full extension.

### Summary Of The Invention

One aspect of the invention provides a prosthetic knee cage comprising a base unit formed by spaced from each other first and second side members interconnected by a connecting element, so as to form an operational channel therebetween. The platform is adapted for pivotal cooperation with the base unit. The adjustment device is provided for adjustment of pivotal movement of the platform relative to the base unit. The adjustment device is situated within an interior proximal region of the base unit and includes a support bar movably arranged relative to the anterior proximal region of the base unit. The adjustment device further includes a support element interconnecting anterior proximal regions of the first and second side members. The support bar is adapted to receive a resilient member.

As to another aspect of the invention, position of the support bar relative to the support element is adjusted by means of adjustment members adapted for threadable engagement with the support bar. Position of the support bar and resilient member relative to the support element can be adjusted by means of rotation of the threadable members within the support element.

As to a further aspect of the invention, the platform consists of a substantially flat table with first and second side plates spaced from each other and extending downwardly therefrom. The platform is formed with an anterior and posterior pivotal mount units separated by the pivotal axle.

An important feature of the invention is that the pivotal axle of the platform is placed well posterior within the structure of the knee cage. Forward or anterior movement of the platform can be adjusted through the use of the adjustment mechanism. Thus, as the platform is allowed to tilt further in the anterior direction, the attached socket including a limb member therefore is placed in a further extended position relative to the pivotal axle. This brings the gravity center of a patient body even further in the anterior direction relative to the pivotal axle.

The preferred embodiment of the knee cage allows for approximately between 20° to 30° of angular adjustment of the platform. Position of the platform perpendicular to the center of gravity is a neutral position. The support bar or stop of the adjustment device can be lowered, so that the platform can achieve inclination or tilt of about 10° - 15° from the horizontal in the anterior direction. In adjusting the platform of the cage in this manner, the weight line passing through the gravity center of a body is allowed to be shifted in the anterior direction relative to the knee center, therefore making the cage and prosthetic knee of the invention inherently stable, just by manipulating the weight line. This occurs without using any locking mechanisms that are dependent upon the system of hydraulics.

As to still another aspect of the invention, a prosthetic device is provided comprising an upper prosthetic limb member, a lower prosthetic limb member, a hydraulic cylinder and a gas cylinder independent from said hydraulic cylinder. The hydraulic cylinder has a hydraulic piston movably disposed thereinside so as to form a hydraulic chamber between an interior of the hydraulic cylinder and the hydraulic piston. A first connecting element extends outwardly from the hydraulic piston. A gas cylinder is also provided having a gas piston movably disposed thereinside, so as to form a gas chamber between an interior of the gas cylinder and the gas piston. A second connecting element extends outwardly from the gas piston. The first and second connecting elements are movably associated with the upper prosthetic limb member, in such a manner that the hydraulic and gas chambers are responsive to operation of the prosthetic leg.

As to a still further aspect of the invention, there is a valve arrangement associated with the gas chamber. Movement of the hydraulic piston toward the lower prosthetic limb member results in expansion of the gas chamber, causing the gas entering the gas chamber through the valve gas arrangement. Upon movement of the hydraulic piston away from said lower prosthetic limb member the gas piston is directed toward the lower prosthetic limb member diminishing the gas chamber. This motion is resulted in compression and discharging the gas from the gas chamber through the gas valve arrangement.

As to still another aspect of the invention, a pressure generated by a resilient bladder forces the hydraulic fluid into the hydraulic cylinder causing motion of the hydraulic piston toward the upper prosthetic member and motion of the gas piston toward the lower prosthetic limb member.

As to a still further aspect of the invention, a pivotal platform is interposed between the upper prosthetic limb member, the hydraulic cylinder and the gas cylinder in such a manner that the first and second connecting elements are pivotally connected to the platform. The platform is pivotal about a pivotal axle situated within a central region of the device. The second connecting element is pivotally connected to the platform through an anterior pivotal mount disposed anterior to the pivotal axle, whereas the first connecting element is pivotally connected to the platform by means of a posterior pivot mount disposed posterior to the pivotal axle.

### Brief Description Of The Drawings

FIG. 1 is a semi-perspective view of an adjustable prosthetic knee cage having a pivotal platform adapted to accommodate in line piston arrangement;
FIG. 2 is another perspective view thereof;
FIG. 3 is an exploded perspective view thereof;
FIG. 4 is a side elevational exploded view thereof;
FIG. 5 is a side elevational assembled view thereof;
FIG. 6 shows the adjustable knee cage of FIGs. 1-5 in an anterior tilt position;
FIG. 7 shows the adjustable knee cage of FIGS 1-5 in a posterior tilt condition;
FIG. 8 is a front elevational view of the adjustable knee cage of FIGs. 1-5;
FIG. 9 is a section view according to section line 9-9 of FIG. 5;
FIG. 10 is a section view according to section line 10-10 of FIG. 8;
FIG. 11 is a semi-perspective view showing an adjustable knee cage having a pivotal platform adapted to accommodate a dual piston arrangement;
FIG. 12 is another semi-perspective view thereof;
FIG. 13 shows a semi-perspective exploded view thereof;
FIG. 14 is a side elevational exploded view thereof;
FIG. 15 a side elevational view showing a neutral position of the platform;
FIG. 16 is a side elevational view showing anterior tilt of the platform;
FIG. 16A is a schematic view illustrating application of the principles of the invention;
FIG. 16B is another schematic view illustrating principles of the invention;
FIG. 16C is a view illustrating another embodiment of the invention;
FIG. 16D is a diagram illustrating principles of the invention;
FIG. 16E is another diagram illustrating principles of the invention;
FIG. 17 is a side elevational view showing posterior tilt of the platform;
FIG. 18 is a front elevational view of the adjustable knee cage of Figures 11- 15;
FIG. 19 is a section view according to section line 19-19 of Figure 15;
Figure 20 is a section view according to section line 20-20 of Figure 18.
FIG. 21 is a diagrammatic representation generally illustrating an above-knee prosthetic leg incorporating the prosthetic knee cage and a knee device of the invention;
FIG. 22 is one side elevational view of the prosthetic knee device of the invention;
FIG. 23 is another side elevational view of the prosthetic knee device; FIG. 24 is a further side elevational view of the prosthetic knee device showing a cover being removed;
FIG. 25 is a partial section view showing one position of the prosthetic knee device;
FIG. 26 is a partial section view showing another position of the prosthetic knee device;
[044] FIG. 27 is a partial section view showing a further position of the prosthetic knee device;
FIG. 28 is a rear elevation view of the prosthetic knee device;
FIG. 29 is a front elevational view of the prosthetic knee device; and
FIG. 30 is a partial sectional view showing in line prosthetic knee device in combination with the knee cage of the invention.

### Description Of The Preferred Embodiment

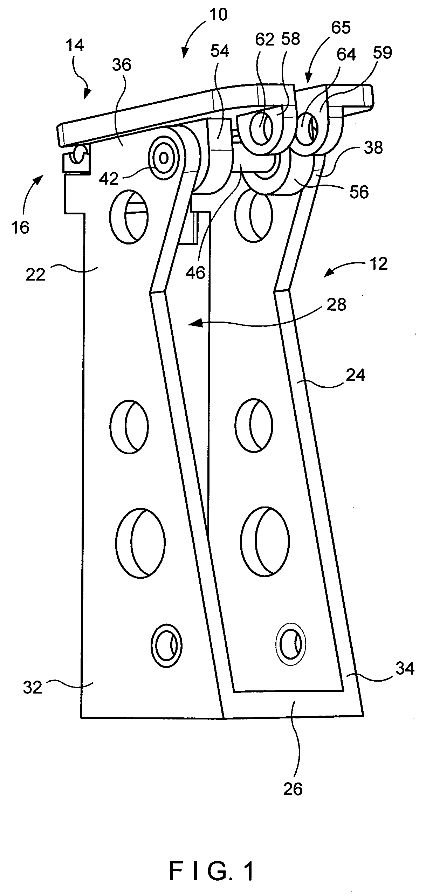

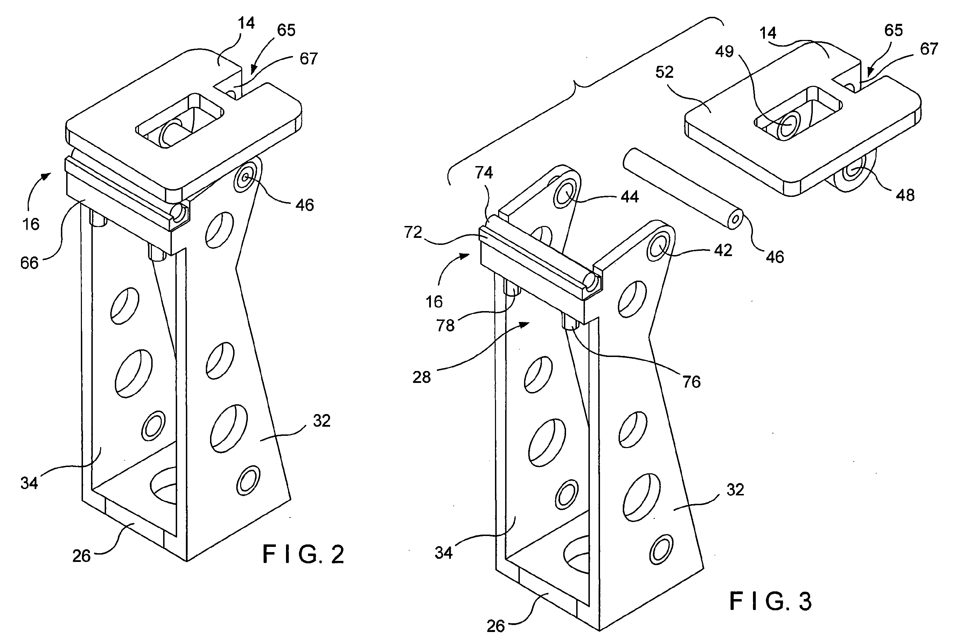

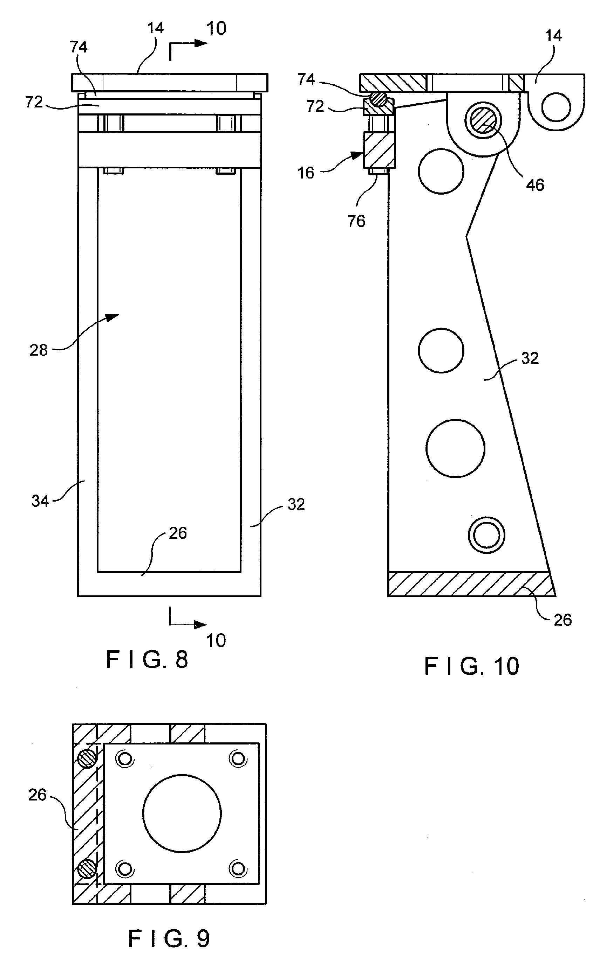

Turning now to Figs. 1-10 illustrating a first embodiment of the adjustable prosthetic knee cage of the invention. The knee cage **10** typically consists of a base unit **12**, a platform **14** and an adjustment mechanism **16**. The adjustment mechanism **16** is located at a front or anterior region of the cage with the apertures **42**, **44** situated at a rear or posterior region thereof. A U-shaped base unit **12** is formed by spaced from each other first side member **22** and a second side member **24** which are interconnected by a connecting element **26**, so as to form an operational channel **28** therebetween. In the preferred embodiment of the invention the first and second side members **22**, **24** are in the form of substantially flat plates extending between their respective distal parts **32** and **34** and proximal parts **36** and **38**. Pivoting apertures **42**, **44** are formed within the proximal region of each side member, so as to receive an axle **46** providing a pivotal connection between the base unit **12** and the platform **14**. The pivotal apertures **42**, **44** and the axle **46** are situated well posterior within the cage. In the assembled condition of the knee cage the pivotal axle **46** passes through the bearing unit accommodated by the pivotal apertures **42** and **44** and also passes through bearings formed within respective pivotal openings **48**, **49** of the platform **14**.

The platform **14** is adapted for movable cooperation with a proximal region of the base unit **12** and consists of a substantially flat top table **52** with first and second side plates **54** and **56** extending downwardly therefrom. In the preferred embodiment of the invention, the side plates **54**, **56** are substantially flat and spaced from each other so as to be adapted for pivotal motion substantially within the proximal region of the operational channel **28** in the vicinity of the inner surfaces of the proximal parts **36** and **38** of the respective first side member **22** and the second side member **24**. Upon the platform **14** being properly received within the operational channel **28**, the pivoting apertures **42**, **44** and the pivoting openings **48**, **49** are adapted for passage of the pivotal axle **46** therethrough.

The platform of the adjustable prosthetic knee cage illustrated in Figures 1-**10** is formed with a single pivot mounting unit **65** which is adapted to pivotally receive a single connecting rod of the prosthetic knee unit. The pivotal mounting unit **65** includes a mounting recess **67** formed within the posterior rear region of the platform **14** and spaced from each other by the mounting recess **67** the first and second supporting elements **58**, **59** which extend outwardly from the bottom posterior area of the platform **14**. As will be discussed later in the application, one connecting element or piston rod of the prosthetic knee unit is adapted to be received within recess **67**, between the first and second supporting elements **58** and **59**. In this manner a pivotal member passes through the apertures **62** and **64** formed within the respective supporting elements, so as to pivotally accommodate the connecting element therebetween.

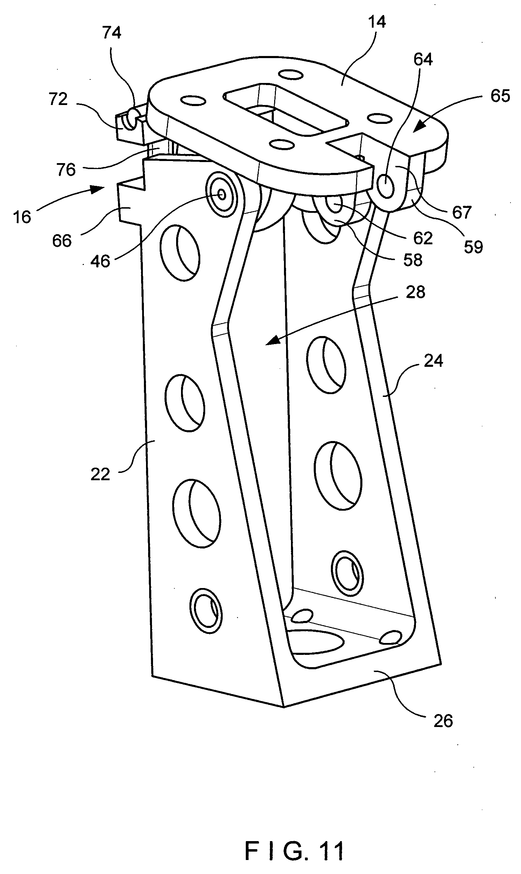

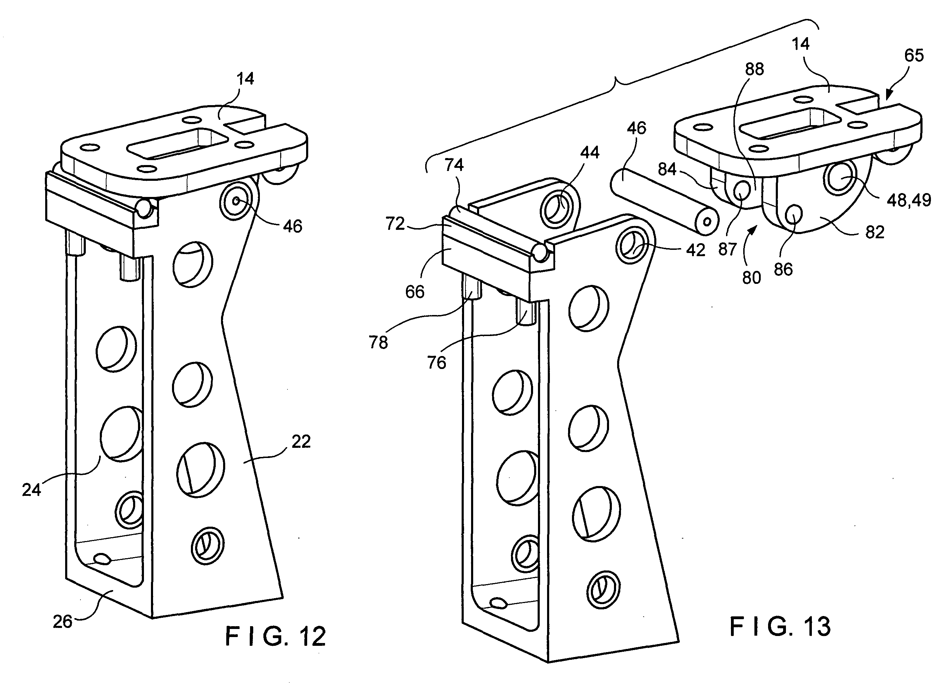

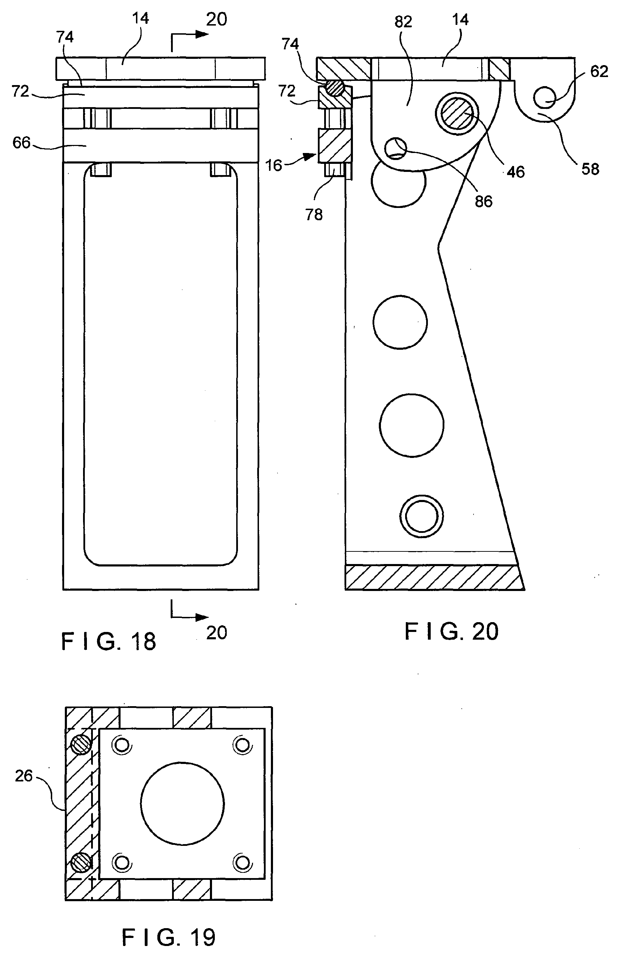

The embodiment of the adjustable prosthetic knee cage illustrated in Figures 11-20 is in many respects similar to the previously discussed embodiment of FIGs. 1-10. However, as best illustrated in at least Figures 13, 14 and 20, the platform **14**, in addition to the posterior mounting unit **65** is formed with an anterior mounting unit **80**, which includes anterior supporting elements **82** and **84** extending outwardly from the bottom area of the platform **14**. The central regions of the anterior supporting elements **82** and **84** are formed with pivoting openings **48** and **49** respectively adapted for passage of the pivotal axle **46**. The anterior region of the anterior supporting elements is formed with apertures **86** and **87** adapted to receive the respective pivotal member. In operation a space **88** between the anterior supporting elements **82** and **84** is adapted to pivotally accommodate another connecting element or piston rod of the prosthetic knee. It will be discussed later in the application that in the fully assembled condition of the prosthetic knee, the platform **14** is supported by the axle **46** and is pivotally mobile with respect to the side members **22** and **24**. Furthermore, one piston rod is pivotally accommodated within the posterior mounting unit **65** between the posterior supporting elements **58** and **59**, whereas another piston rod is pivotally accommodated within the space **88** of the anterior mounting unit **80** between the anterior supporting units **58** and **59**.

Mounting holes can be provided within the table 52 and connecting element **26** which are adapted to accept existing components which are common in the prosthetic industry.

An adjustment mechanism **16** is situated in the anterior proximal region of the knee cage and consists of a support bar **72** or a stop which is movably arranged with respect to the support element **66** interconnecting the anterior proximal regions of the first and second side members **22** and **24**. The support bar **72** is adapted to receive a resilient member **74**. Position of the support bar **72** with respect to the support element **66** is adjusted by means of adjustment elements **76** and **78**. In the preferred embodiment of the invention the adjustment elements **76** and **78** are in the form of threadable members or screws. Thus, the position of the support bar **72** and the resilient member **74** can be adjusted by means of rotation of the threadable elements **76** and **78**. As the threadable members are turned in one direction the support bar **72** is elevated with respect to the base unit **12** in general and specifically with respect to the support element **66**. In this position the downward motion of the anterior portion of the adjustable platform **14** in general, and the table **52** in particular, is restricted.

Although the adjustment mechanism utilizing the threadable adjustment elements **76** and **78** have been described hereinabove, it should be understood that the adjustment mechanism utilizing any conventional means of elevating or lowering the support bar **72** with respect to the support element **66** is within the scope of the invention.

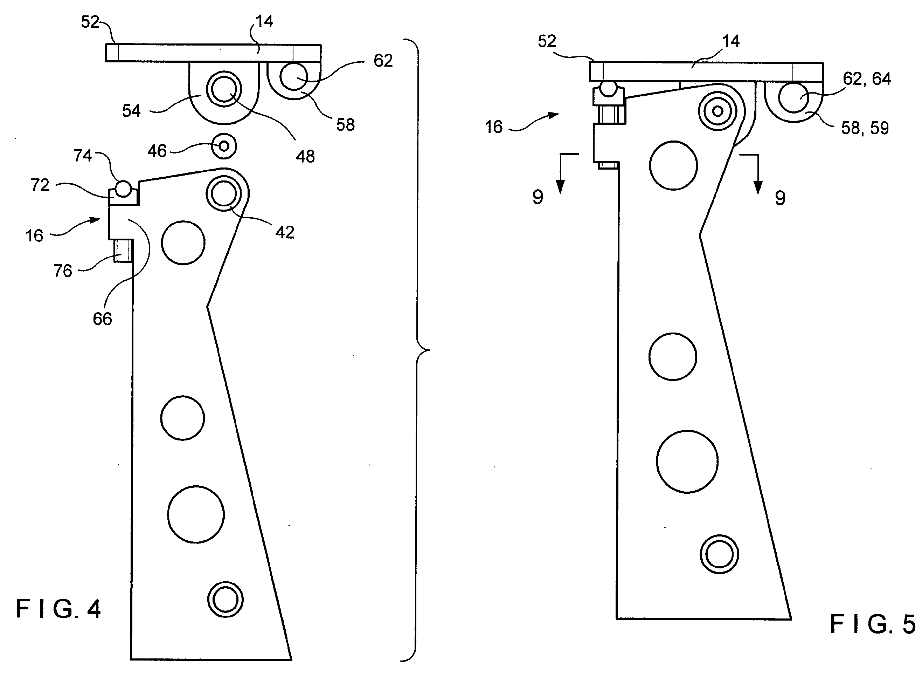

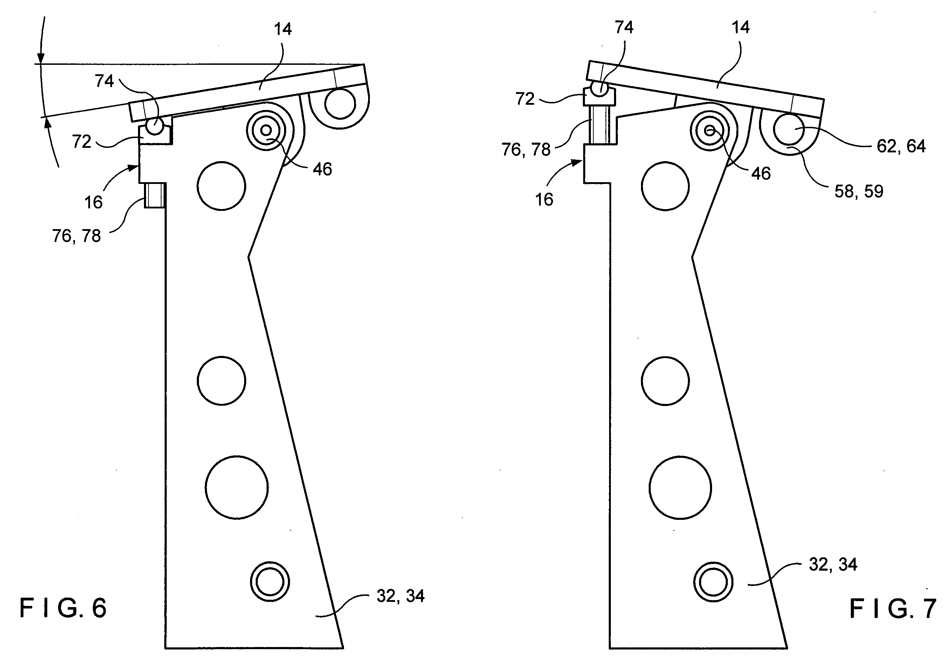

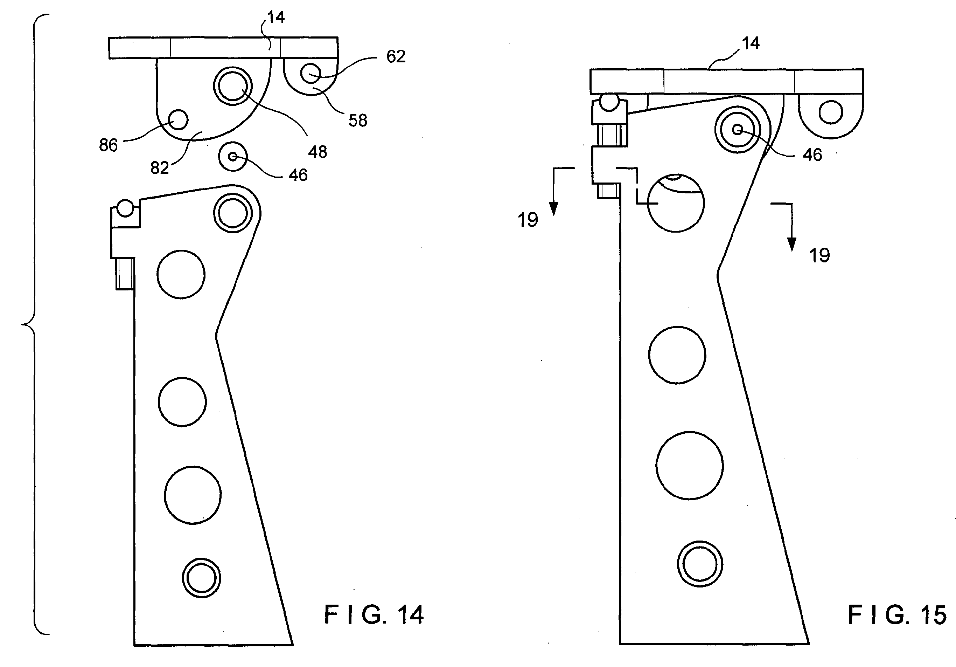

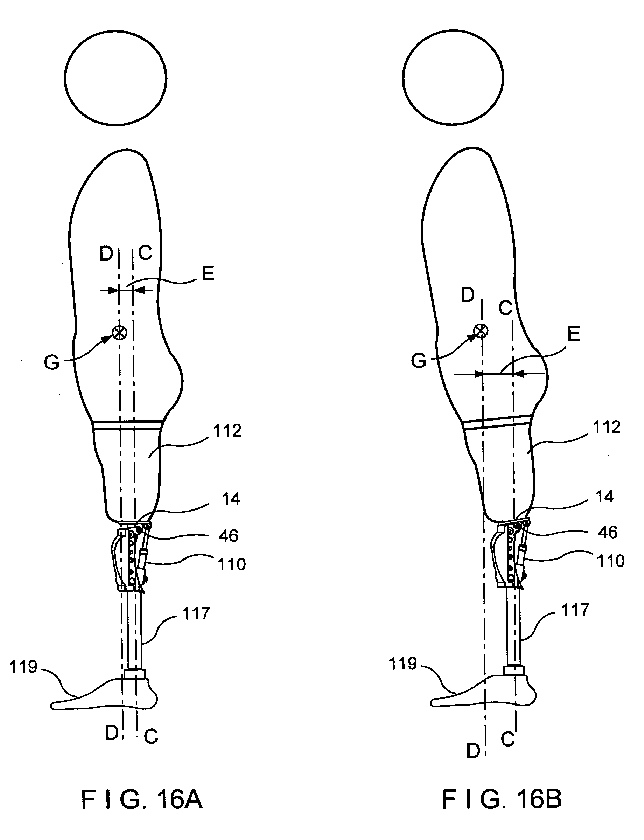

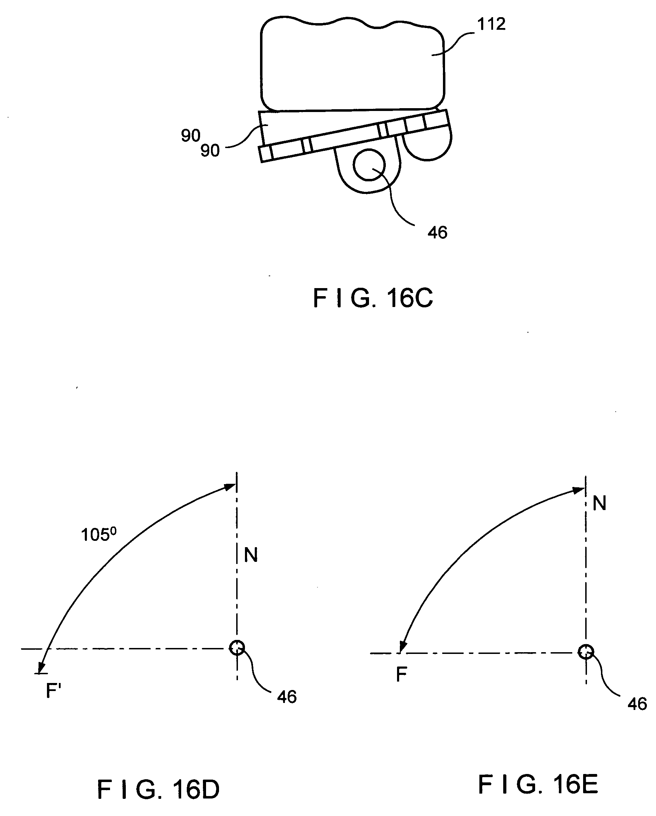

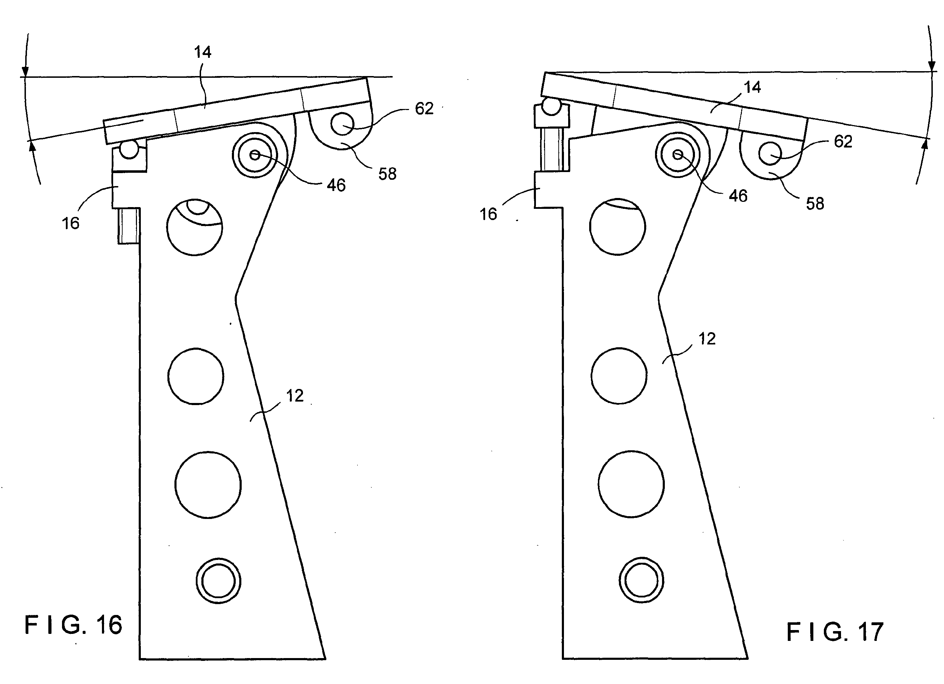

In operation of the knee cage, upon elevation of the support bar **72** by the adjustment mechanism **16** the platform **14** including the table **52** is prevented from reaching its full downward position. Thus, the slant of the platform **14** is regulated by the height or elevation of the bar **72** and resilient member **74**. The reason for the anterior tilt or the over center nature of the cage is as follows. As the knee is brought to full extension because of the placement of the foot, there is a moment of instability when the knee is fully extended and not being controlled by the prosthetic knee mechanism itself. In the invention, the knee cage **10** allows the knee to become stable by allowing the weight line passing through the gravity center of a person's body to be brought well anterior to the location of the pivotal axle **46**. If the platform **14** including the table **52** is allowed to go beyond the horizontal level (see FIGs. 5 and 15) and to reach an anterior slanting position (see FIGs. 6 and 16), the weight line passing through the gravity center of a person's body is moved even further in the anterior direction relative to the axle **46**. By moving the weight line and the gravity center of a person's body anterior to the axle **46**, the invention prevents the knee cage from folding backwards, or to go into the flexion. This position is named hyper-extension. In the extension, the anterior sloping position of the platform **14** is achieved (see FIGs. 6 and 16), rendering the knee to become stable. This is because, the weight line is situated to be anterior to the location of the axle **46**. The invention allows to place the weight line passing through the gravity center of a person's body to be anterior to the pivotal axle **46** of the knee cage. The more anterior the center of gravity is brought relative to the axle **46**, the more stable the prosthetic knee becomes. This aspect of the invention is more specifically illustrated in FIGs. 16A and 16B in which the axis C-C passes through a pivotal axle **46** and the weight line D-D passes through the gravitational center G of a person's body. Referring now to FIG. 16A, illustrating a vertical orientation of a body with substantially horizontal position of the platform **14**. In this instance the distance E between the axis C-C and the weight line D-D is relatively small. However, when the body moves forward, as illustrated in FIG. 16B, the gravity center G and the weight line D-D moves further in the anterior direction from the axis C-C. This move is reflected in the increased distance E, indicating that the weight line is shifted anterior to the pivotal axle **46**.

Another feature of the invention is illustrated in FIG. 16C, which is applicable when a patient is not able to have enough hip extension to accommodate anterior slanting position or forward inclination from a horizontal position. In such instance, a wedge-shaped member **90** can be interposed between the platform **14** and the socket **12.** The member **90** allows the patient's limb to become oriented substantially horizontally or at the angle of 180° relative to the ground, while also allowing the platform **14** to assume the beneficial anterior inclination.

There are situations where the center of gravity of a patient's body is not significantly shifted in the anterior direction relative to the axis of rotation or axle **46** because of the orientation of the center of gravity being closer to the center of rotation of the platform **14**. The advantage gained by having the anterior inclination of the platform **14** can offset the inability of bringing the weight line anterior. This occurs in view of the increasing the amount of torque necessary to reach the null point. Considering how much energy it takes to move a certain mass at a certain fulcrum distance from that mass, if the mass must be moved in an arc moment in relation to the fulcrum, it may be necessary to move the tilted version of the platform about 105°, where the movement at the level version with the platform being parallel to the ground, it takes only about 90°. Referring now to FIG. 16D, where we can see the resting point F' to the null point of 105° in relation to the axis or the center of rotation of the unit. In FIG. 16E shows the axis being at a level with the center of rotation, rotating to 90° before the null point N is reached in relation to the axle or center of rotation.

The center of gravity of a person's body is located at approximately lumbar to at or about the height of the navel. The center of rotation as illustrated in FIGs. 16D and 16E passes through the axle **46**. When the socket is placed at a level attitude in relation to the ground with the knee unit at a level position, it is possible to gain as much as 1.5" in terms of the anterior shifting of the center of gravity in relation to the center of rotation or axle **46**. In dealing with manipulating the weight line passing through the gravity center, when the tilt of the platform is forced to an anterior position, the center of gravity is forced to be in a more anterior or forward position. This, brings the weight line anterior to the center of rotation or axle **46** by an increase factor.

Raising or lowering the support bar or stop **72** of the knee cage allows the prosthetic knee to flex beyond 180° or less than 180°. When the flexion is beyond 180° or when the anterior slanting position is reached, additional stability is insured. On the other hand, when the flexion is less than 180°, or the posterior slanting position is accomplished, the controlled position of the knee unit is insured. This condition might be desirable for some patients having extremely long limbs. In the elderly population, and in the majority of the amputee population, increased stability not only becomes desirable for safety, but it is also necessary to insure that these patients do not fall. Some patients have knee flexion contractures. This occurs due to shortening of the ligemental structures and the tendon structures in their leg, causing the patients to have less than full extension, in terms of their hip flexing muscles. In the invention this condition can be accommodated by adjusting the knee cage in such a way so as to accommodate the knee flexion contracture. The stability of a patient can be maintained by adjusting the ankle of the tibia in relation to the foot. This brings the weight line anterior in relation to the femoral socket or the trans-femoral socket in relation to the center of gravity of the body.

Although the knee cage of the invention is described with reference to specific prosthetic knee units, it should be noted that the prosthetic knee cage of the invention is not limited to applications with the discussed knee systems. The knee cage can be used with other prosthetic knee systems, as long as such systems fit into the confinement of the elements of the cage.

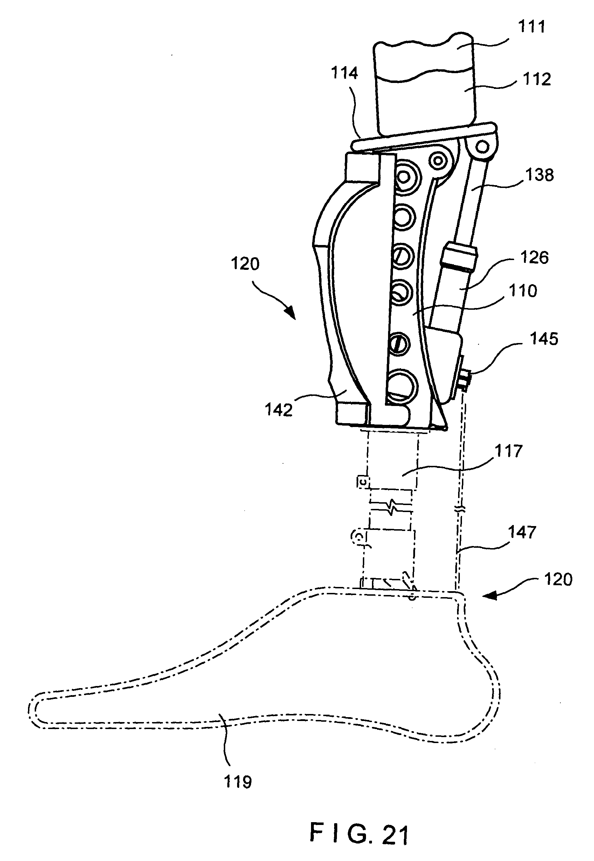

Referring now to FIG. 21, illustrating the above-knee prosthetic leg which includes the prosthetic knee cage and the prosthetic knee. The prosthetic leg is designated generally by the numeral **120** and includes a socket or an upper prosthetic limb member **112** adapted for receiving a leg stump **111** of an amputee. The socket **112** is coupled by a conventional arrangement to an adjustable platform **114** provided at the top portion of the knee cage **110**, which can be provided with a removable cover **142**. As it has been discussed substantially in this application, the platform **114** is adapted for pivotal motion relative to the base of the cage. A pylon **117** at one end is coupled to the bottom of a knee cage **110** and at the other end is coupled to a prosthetic foot **119**. The pylon **117** and prosthetic foot **119** define a lower prosthetic limb member **120**.

The prosthetic knee assembly of the invention is positioned within the cage **110** and consists of a double cylinder arrangement **122** and a control valve-bladder sub-assembly **124,** etc. These elements form a part of independent from each other a hydraulic system **121** and a pneumatic system **123**. With respect to the connection with the platform **114**, a gas or air cylinder unit **128** and a hydraulic cylinder unit **126** are arranged tandemly on either side of the axle **146** of the prosthetic knee cage **110**.

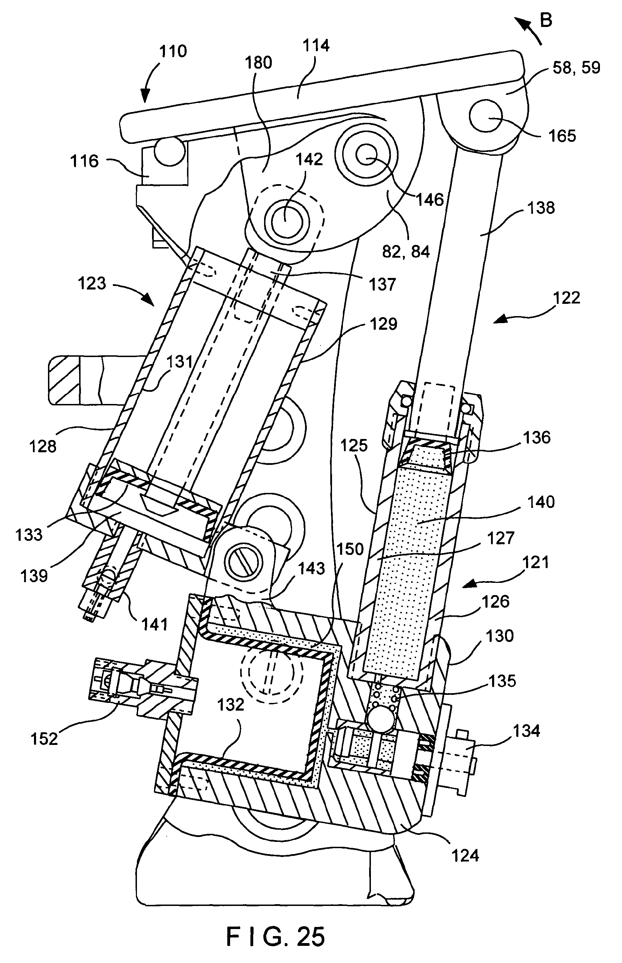

The hydraulic system **121** includes a main body **130** adapted to receive a bladder **132** and a cam valve **134** operationally connected to the hydraulic cylinder **126**. A cartridge check valve **135** is situated within the main body directly below the output region of the hydraulic cylinder. As best illustrated in FIGs. 25, 26 and 27, the hydraulic cylinder **126** is formed by a substantially cylindrical wall **125** having an inner surface **127** and contains therein a piston **136** which is displaced by a suitable fluid which may be, for purposes of illustration, a silicone oil. A hydraulic chamber **140** is formed within the inner space of the cylinder **126** between its bottom and the piston **136**. A first connecting element or hydraulic piston rod **138** at one end attached to the piston **136**, and at the other end is pivotally connected to the platform **114** of the cage at the posterior pivot mounting **165**. The main body **130** is coupled to the cylinder **126** and supports a flow control valve.

The main body **130** is also formed with a chamber adapted to receive a substantially horizontally disposed rotary cam valve which is sealed around its outer most circumference by a lip seal.

The pneumatic system **123** includes the gas cylinder unit **128** which is formed with a substantially cylindrical housing **129** having at least an interior surface **131**, with the gas piston **133** movably position thereinside. A second connecting element or gas piston rod **137** is at one end thereof pivotally connected to the platform **114** of the cage at an anterior pivotal mount **180**, whereas the other end is attached to the piston **133**. A gas chamber **139** is formed within the inner space of the gas cylinder **128** between the bottom portion of the cylinder and the piston **133.**

One of the main functions of the pneumatic system **123** including the gas cylinder **128** is to minimize the effect of the terminal impact and to slow down motion of the leg during swing phase. As best illustrated in FIGs. 25-27, the gas cylinder **128** is movably and/or pivotally coupled to the main body **130** of the hydraulic unit via a mounting arrangement which includes mounting plates **143** positioned on either side of the main body.

The dual piston arrangement is pivotally associated with the knee cage assembly previously described with respect to Figures 11- 20. It is best illustrated in at least FIGs. 25- 27 that the gas piston rod **137** is pivotally connected through the anterior pivot mount **180** which is disposed anterior and below the central pivotal axle **146**. On the other hand, the hydraulic piston rod **138** is pivotally connected to the posterior pivot mount **165** which is disposed posterior and above the central pivotal axle **146**. As discussed hereinabove, the platform **114** is pivotally supported by the central pivotal axle **146** within the base of the knee cage.

A valve arrangement or gas valve **141** is disposed within the wall of the gas cylinder **128**, so as to provide communication between the gas chamber **139** and outside environment. In the preferred embodiment of the invention an ambient air is used for the operation of the gas cylinder **128**. Nevertheless, it should be noted that utilization of any suitable gas for the same purpose is also contemplated. The valve arrangement **141** can be adjusted to regulate flow of gas out of the gas chamber **139**. During movement of the gas piston **133** in one direction the gas valve arrangement **141** is adapted to controllably discharge the gas accumulated within the gas chamber **139**, whereas during the movement of the piston **133** in the opposite direction it operates as a check valve introducing air or gas into the expanding gas chamber.

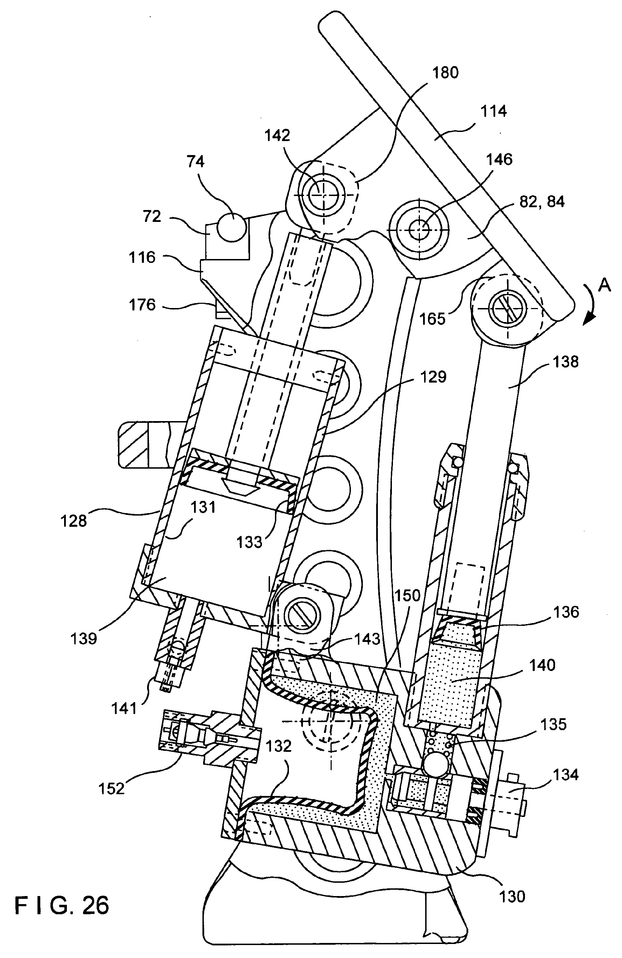

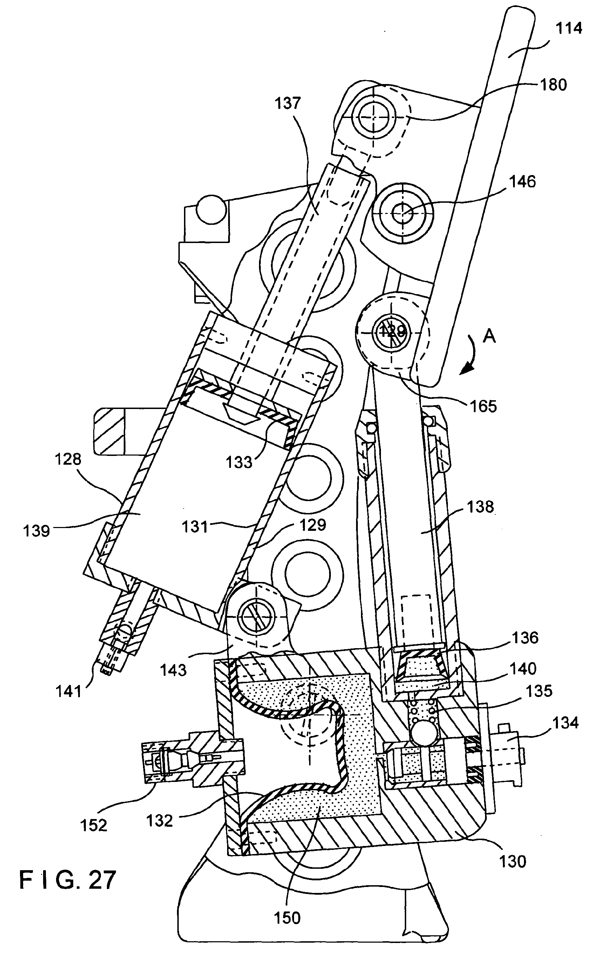

The prosthetic knee of the invention is capable of achieving approximately a 126° angle in the movement between the fully flexed position to the fully extended position. The fully flexed position, as illustrated in FIG. 27, corresponds to the situation where the knee is folded or a patient achieving a kneeling position in which the gas chamber **139** is filled with gas. As the prosthetic knee moves from the fully flexed position to the fully extended position (see FIGs. 26 and 27) the gas trapped within the gas chamber is controllably discharged by means of the gas valve **141** regulating the speed and intensity of the extension cycle.

Operation of the check valve **135** and the rotary cam **134** do not form an essential part of the hydraulic system and have been in full detail disclosed by U.S. Patent 5,779,735 which the present application incorporates by reference. Similar to the prosthetic knee disclosed by U.S. Patent 5,779,735, a bladder membrane **132** is situated within the bladder chamber **150** which includes a bladder valve **152**. The valve **152** is adjustable for varying bladder compression, flexion resistance and extension drive. The bladder **132** is affected by the operation of valves situated within the main body **130**, so as to be compressed for storing all of the required energy. The bladder is capable of storing the kinetic energy for the return cycle or the upward motion of the hydraulic piston **136**. This enables the hydraulic fluid to be moved from the accumulation bladder chamber **150** into the hydraulic chamber **140** to have the required resistance within the cylinder **126** when the bladder collapses. Such action accumulates and stores the kinetic energy required for the operation of the knee unit of the invention.

An accumulation chamber **150** accommodates the displaced oil. As the oil is displaced during a compression stroke, pressure is exerted on the bladder membrane **132** allowing it to compress the gas situated thereinside and accept the displaced oil. Pressure is increased on the inner area of the bladder membrane **132**, thus storing enough kinetic energy to power the return cycle and return of the piston **136** to the extended position. The air valve **152** communicating with the airside of the bladder chamber can adjust the level of kinetic energy. The force necessary to compress and extend the piston **136** and piston rod or connecting element **138** is adjustable by increasing or decreasing the amount of air pressure in the bladder cylinder **150**. During operation of the knee the kinetic energy is stored in the bladder capable of driving the piston **136**.

A cartridge-type ball check valve **135** is placed between the hydraulic chamber **140** and the bladder chamber **150**. To stop the flow of fluid from the cylinder **126** to the bladder chamber **150** a rotary cam **134** associated with the ball check valve **135** is provided. As the cam is rotated, the ball is lifted or lowered into the ball seat either allowing or not allowing passage of fluid through the ball seat to the accumulation chamber. Free oil flow is allowed back into the cylinder **126** due to the kinetic energy stored in the compressed air/gas in the bladder chamber **150** pushing on the bladder **132** to return the oil to the hydraulic chamber **140** through the check valve **135** to the cylinder returning the cylinder to the extended position.

The hydraulic chamber **140** is pressurized through the downward motion of the piston **136**, so as to allow the hydraulic fluid to pass through the cartridge valve **135** only when the cam lobe is in a position to lift the ball out of its seated position in the cartridge valve **135**, thereby allowing fluid to pass around the cam. As the fluid passes around the cam when the cam is in the open position, the hydraulic fluid then enters through an orifice in the back of the cam chamber. Through that orifice the hydraulic fluid is allowed to pass through the hole just posterior to the cam housing into an accumulated chamber. The valve 135 situated above the cam also operates as a check valve. This means that it has a spring associated with the ball valve mechanism. Thus, as the pressure on the bladder side of the valve **135** is greater than the pressure on the cylinder side, the bladder pressure pushes the fluid past the ball valve **135** back into the cylinder **126** returning the plunger **136** and the rod **138** to their original extended position.

In operation, as the platform **114** of the knee cage is allowed to pivot posteriorly or downwardly, as illustrated by the arrow A in FIGs. 26 and 27, the hydraulic rod **138** and the hydraulic piston **136** are moved compressing the fluid and enabling passage thereof through the channels of the main body **130**, so as to compress the bladder **132**. In this situation the gas piston **133** reaches fully extended and the hydraulic piston **136** is retracted (see FIG. 27). After the finish of the gait cycle and during swing phase, the accumulated kinetic energy in the bladder **132** acts to return the piston **136** of the hydraulic cylinder to its extended position. In this motion the hydraulic rod **138** and the platform **114** of the knee cage are returned to their original position. If this motion is not properly controlled, it occurs too fast resulted in a loud sound of engaging mating surfaces. The gas cylinder **128** provided with the adjustable valve mechanism **141** which controllably discharges the gas out of the gas chamber **139** into the atmosphere. This arrangement prevents this undesirable action and regulates the speed of the swing phase and the terminal impact. The amount of generated kinetic energy can be adjusted by means of the gas valve **152** associated with the bladder. If more extension is needed, the gas pressure in the bladder **132** should be increased. The normal operating pressure inside the bladder is between 60 and 150 pounds, according to the activity level of the patient.

To summarize the above, as the kinetic energy generated by the compression of the bladder **132** forces the fluid into the hydraulic chamber **140** causing the hydraulic piston **136** to be pushed outward or toward the upper prosthetic limb member, resulted in the pivotal motion of the platform **114** of the knee cage upward (as illustrated by the arrow B in FIG. 25). This motion places the gas piston **133** into a retracted or downward position, causing compression of the gas in the gas cylinder **128**. Significantly, initially during the downward motion of the piston **133** the gas is pressurized in the gas chamber **139**. This occurs in view of the compressible nature of the gas. As the gas or air is being further compressed, the restrictive forces make it more difficult for the piston **133** to reach the bottom of the gas cylinder **128**, resulted in minimizing the terminal impact. The adjustable bleeding nature of the gas valve **141** positioned at the bottom of the gas cylinder **128** is capable of releasing air in a controlled fashion, and to inhibit the rapid and uncontrolled return of the prosthetic knee and the platform of the knee cage to its most forward or downward position. In this manner, a resultant damaging force and loud noise are minimized or completely eliminated.

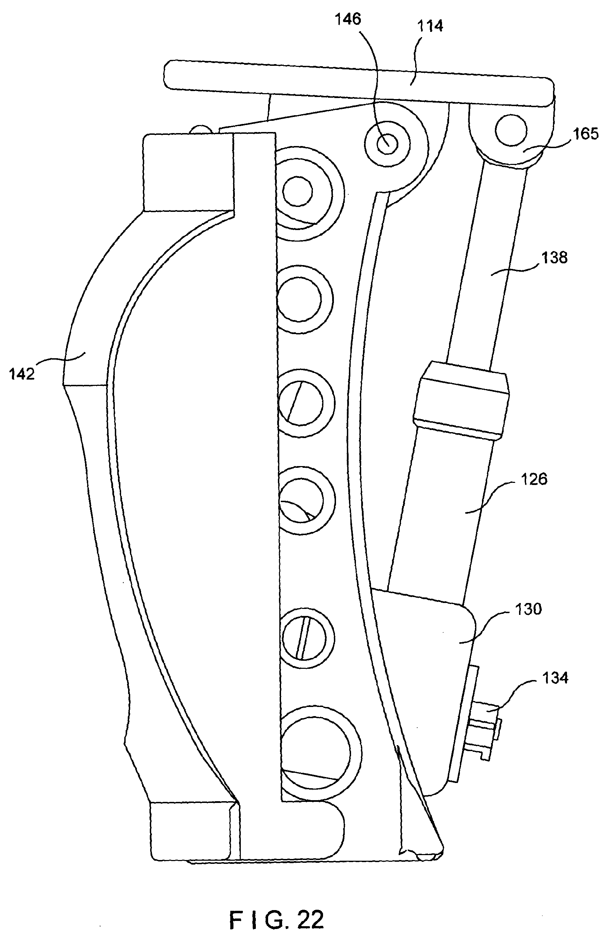

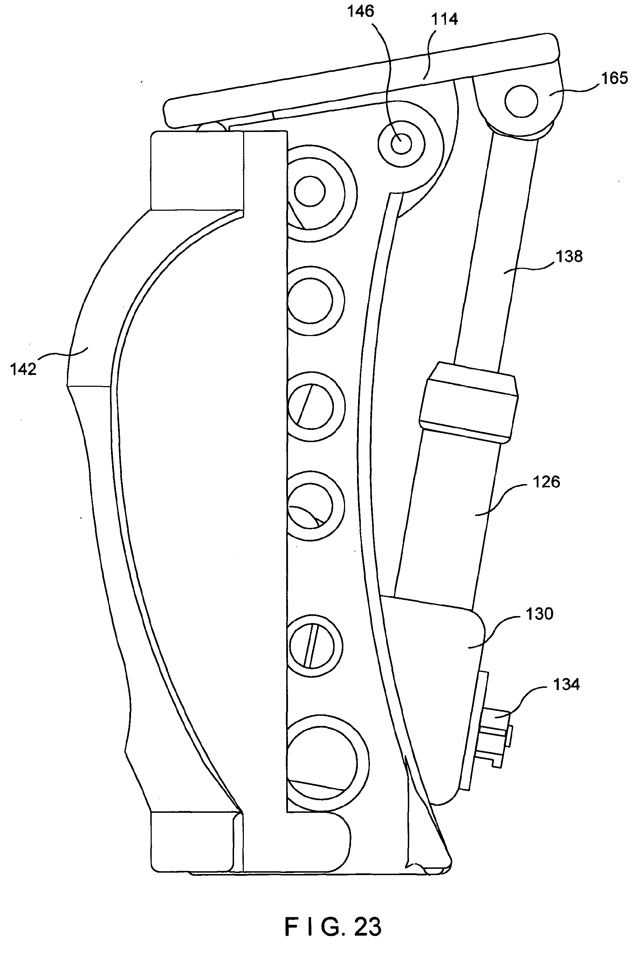

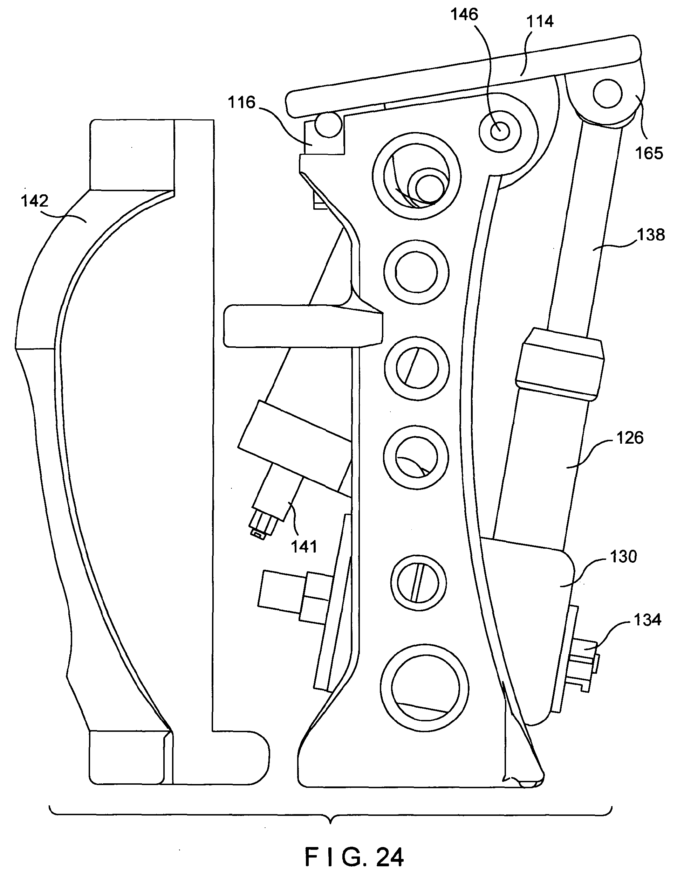

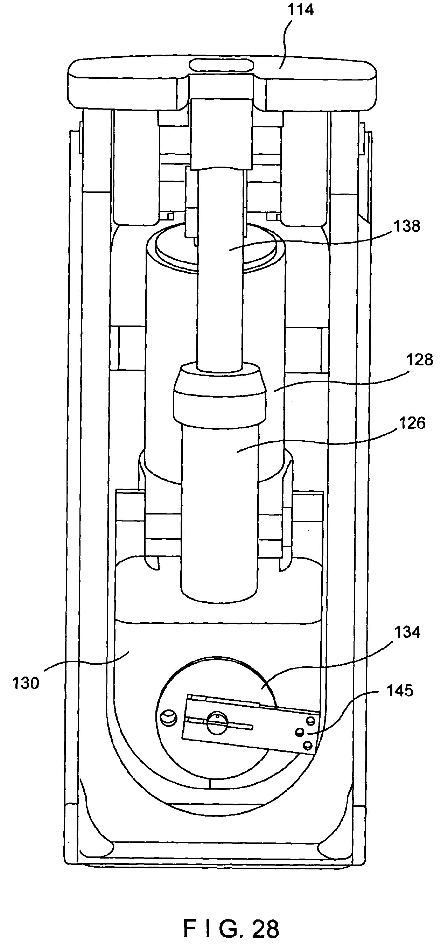

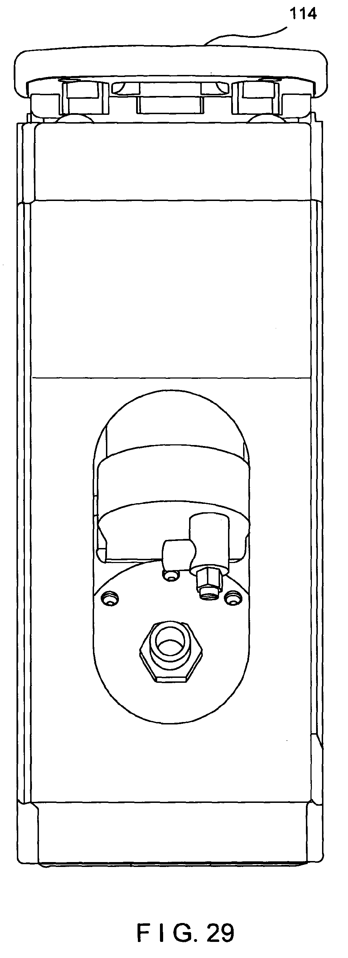

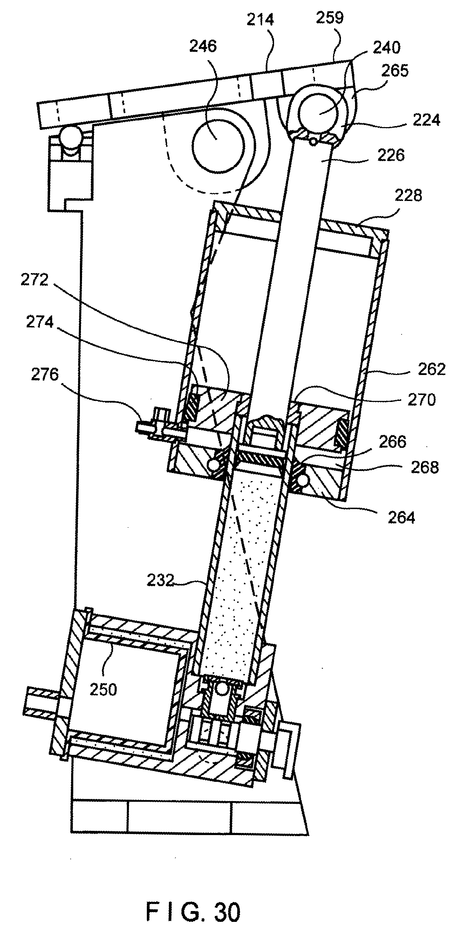

The adjustable prosthetic knee cage which was previously described with reference to FIGs. 1-20 is also adaptable for use with the prosthetic knee having an in line piston arrangement. The structure and operation of this prosthetic knee do not form as essential part of the invention and have been in full detail disclosed by the U.S. patent application S.N 10/278,361 filed October 23,2002, which this application incorporates by reference. Similar to the prosthetic knee having the dual piston arrangement previously discussed with reference to FIGs. 21-29, as illustrated in FIG. 30 the in line piston arrangement also includes a multi piston structure which is movably connected to the platform **214** of the cage by a single pivot mount **224**, which is situated in the platform **214** posterior to the central axle **246**.

In the prosthetic knee having the in line piston arrangement a gas piston cylinder **262** is provided outside the hydraulic cylinder **232**. The bottom of a gas cylinder **262** is formed with an upwardly facing lip edge **266**. The cap **270** seals the hydraulic cylinder **232** and also traps the gas between that seal and the bottom portion of the concentric gas plunger **272**. A closing cap **228** is provided at the top of the gas cylinder **262**, so as to fixedly receive the piston rod **226** which extends between the pivot mount **224** and the hydraulic piston **234**. As the platform **214** of the knee cage is moved downwardly, the piston **234** pushes the hydraulic fluid through the system in a manner discussed hereinabove. In this motion, in view of the permanent connection between the piston rod **226** and the gas cylinder **262**, the gas chamber **268** is also expanded. This is because the gas cylinder **262** moves up and down in relation to a fixedly positioned gas plunger **272**, **274** that is mounted on the cap **270** of the hydraulic unit. In this motion the gas volume increases, as the platform **214** is tilted or moved downwardly. In this action more gas enters the chamber **268** between the plunger **272**, **274** and the bottom portion **264** of the gas cylinder. When the unit is extended, as a result of the kinetic energy stored in the bladder **240**, the gas accumulated in the chamber **268** is being compressed. In this manner, as the platform **214** of the cage returns to its normal position, the bottom portion **264** compresses the gas against the plunger **272** and traps gas therebetween. As the distance between the bottom portion **264** and plunger **272** decreases, gas is gradually and substantially pressurized. The pressurized gas slows down the swing phase, damping the terminal impact. A gas valve **276** is provided at a lower portion of the gas cylinder **260**. As the gas is compressed in the chamber **268**, it is allowed to escape through the valve **276**. During the initial stages of the swing phase, gas is compressed rapidly in the chamber **268** until the pressure reaches a point where it cannot be compressed rapidly. During the slow rate of compression gas is allowed to escape through the valve **276** and therefore damping swing phase. This arrangement minimizes or eliminates terminal impact, so that there is a proper rate of return for the distal portion of the leg. The gas in the form of air is used as a medium because initial compression is easy until the pressure builds up and the last portion of the travel becomes more difficult. This occurs when the maximum amount of damping power is needed just before the leg becomes fully extended, so that it does not advance too rapidly without causing a resultant klunk.

As indicated hereinabove, the in line piston arrangement of the prosthetic knee is adapted for cooperation with the knee cage which has been previously discussed with reference to Figures 1-10. As best illustrated in Figure 30, the piston rod **226** by means of the pivotal member **240** is movably connected to the posterior mounting unit **265**. The free end of the piston rod **226** is received within the mounting recess **259** between two posterior mounting plates.

The knee cage of the invention adapted for cooperation with the in line system illustrated in FIG. 30 operates in a manner similar to that used with the dual piston arrangement of FIGs. 21-29. As the prosthetic knee cage interacts with the forces that are initiated by the return of the hydraulic fluid into the cylinder **232**, the platform **214** is moved to an upward or substantially horizontal or anteriorly tilted position. When the platform **214** is tilted anterior the knee center can be manipulated to be considerably posterior to the center of gravity of a patient. When the platform **214** is in a level position, the weight of a patient travels approximately ½ to ¾ of an inch anterior to the central axle **246** promoting stability. However, when the platform has an anterior tilt as much as 5 inches of travel can be achieved anterior to the axle promoting extreme stability.

Actuation of the cam valve **134** in the prosthetic knee of the invention is associated with at least one of the following three modalities. One mode of operation is mechanical in nature and operates by providing a connector or cable **147** between the prosthetic knee and a movable ankle or foot **119** (see FIG. 21). When the foot is in planter flexion or when the ankle position allows planter flexion of the foot, the connector or cable **147** pulls on the lever or arm **145** which locks the cam valve **134** into a closed position preventing flexion of the knee. As the leg is brought into a vertical position, the cam valve **134** is placed into an open position, thereby allowing the flow of fluid from the hydraulic cylinder **126** into the bladder chamber **150**.

The prosthetic knee of the invention is also adaptable for operation in the electro-mechanical mode, whereby a switch located on the plantar surface of the foot or the bottom surface of the foot can be activated. Such activation elicits a response from a survo to pull the arm **145** into a position in which the foot contacts the floor. The cam valve **45** is allowed to be placed into a position facilitating the drop of the ball into the seat, so as to seal the unit. Such action prevents flow of the fluid through the prosthetic knee leading to complete stoppage of the flexionability of the knee.

A further mode of controlling the knee is by means of myoelectric control. In this mode a signal from the residual musculature, or an electronic signal from the brain of a patient to the residual musculature is amplified and sent to a discriminator circuit. There the signal is analyzed to define the type of signal in terms of its amplitude and duration. After that another discrimination step can be conducted to define a selected response mode or activity such as walking, climbing stairs, descending stairs, seating, etc. The proper placement of the cam valve **134** is elicited by an arrangement utilizing a signal from the discriminator circuit. This provides a measured travel, so as to place the cam valve **134** in a desired position, in order to coincide with the motion that is elicited by the brain. This is necessary to achieve one of the previously discussed activities.

## Claims

1. A prosthetic knee cage, comprising:
a base unit formed by spaced from each other first and second side members interconnected by a connecting element, so as to form an operational channel therebetween;
a platform being adapted for pivotal cooperation with said base unit; and
an adjustment device for adjustment of pivotal movement of said platform relative to said base unit.

2. The prosthetic knee cage according to claim 1, wherein said adjustment device is situated within an anterior proximal region of said base unit, said adjustment device comprises a support bar movably arranged relative to said anterior proximal region of the base unit.

3. The prosthetic knee cage according to claim 2, wherein said adjustment device further comprises a support element interconnecting anterior proximal regions of said first and second side members and said support bar is adapted to receive a resilient member.

4. The prosthetic knee cage according to claim 3, wherein position of said support bar relative to said support element is adjusted by means of adjustment members associated with said support element.

5. The prosthetic knee cage according to claim 4, wherein said adjustment members are in the form of threadable members adapted for threadable engagement with said support bar, so that adjustment of position of said support bar and resilient member relative to the support element is adjusted by means of rotation of said threadable members within said support element.

6. The prosthetic knee cage according to claim 4, wherein upon elevation of said support bar relative to the support element downward motion of an anterior part of the platform is restricted, and upon lowering position of said support bar relative to the support element the downward motion of said anterior part of the platform is increased.

7. The prosthetic knee cage according to claim 1, wherein said platform further comprises a substantially flat table with first and second side plates spaced from each other and extending downwardly from said table, thus facilitating pivotal motion of said platform within a proximal region of the operational channel about a pivotal axle.

8. The prosthetic knee cage according to claim 7, wherein said pivotal axle is centrally located within the platform and said platform is formed with a posterior pivotal mounting unit situated posterior to said pivotal axle.

9. The prosthetic knee cage according to claim 8, further comprising an anterior mounting unit situated within said platform anterior to said pivotal axle, said anterior mounting unit comprises a pair of spaced from each other anterior supporting elements extending outwardly from a bottom portion of the table, an anterior region of the anterior supporting elements is adapted to pivotally receive a connecting element therebetween.

10. The prosthetic knee cage according to claim 1, wherein said platform is capable of achieving an inclination in an anterior direction from a horizontal.

11. The prosthetic knee cage according to claim 10, wherein said anterior inclination is between 10° and 15° from horizontal.

12. A prosthetic device, comprising:
an upper prosthetic limb member;
a lower prosthetic limb member;
a hydraulic cylinder having a hydraulic piston movably disposed thereinside so as to form a hydraulic chamber between an interior of said hydraulic cylinder and said piston, a first connecting element extending outwardly from said hydraulic piston;
a gas cylinder independent from said hydraulic cylinder and having a gas piston movably disposed thereinside, so as to form a gas chamber between an interior of said gas cylinder and said gas piston, a second connecting element extending outwardly from said gas piston; and
said first and second connecting elements being movably associated with said upper prosthetic limb member,
whereby said hydraulic and gas chambers being responsive to operation of said prosthetic leg.

13. The prosthetic device according to claim 12, wherein upon movement of said hydraulic piston toward said lower prosthetic limb member the gas chamber is expanded.

14. The prosthetic device according to claim 12, wherein upon movement of said hydraulic piston away from said lower prosthetic limb member said gas chamber is reduced so as to compress a gas accumulated thereinside.

15. The prosthetic device according to claim 12, further comprising a gas valve arrangement associated with said gas chamber, so that upon movement of said hydraulic piston toward the lower prosthetic limb member and expansion of said gas chamber the gas enters said chamber via said gas valve arrangement.

16. The prosthetic device according to claim 15, wherein upon motion of said hydraulic piston away from said lower prosthetic limb member said gas piston being directed toward said lower prosthetic limb member diminishing the gas chamber, so as to compress and discharge said gas from said gas chamber via said gas valve arrangement.

17. The prosthetic device according to claim 15, wherein said gas valve arrangement is situated within the gas chamber in the vicinity of a bottom portion thereof.

18. The prosthetic device according to claim 16, wherein said gas valve arrangement provides communication between the gas operational chamber and an outside environment and said gas is an ambient air.

19. The prosthetic device according to claim 13, wherein said movement of the hydraulic piston toward said upper prosthetic limb member is caused by a kinetic energy accumulated by a resilient bladder.

20. The prosthetic device according to claim 19, wherein said kinetic energy is in the form of a pressure applied by said bladder to a hydraulic fluid situated within said hydraulic cylinder.

21. The prosthetic device according to claim 20, wherein said pressure generated by said resilient bladder forces said hydraulic fluid into the hydraulic cylinder causing the motion of said hydraulic piston toward said upper prosthetic member and motion of said gas piston toward said lower prosthetic limb member.

22. The prosthetic device according to claim 12, wherein a pivotal platform is interposed between said upper prosthetic limb member and said hydraulic cylinder and said gas cylinder in such a manner that said first and second connecting elements are pivotally connected to said platform.

23. The prosthetic device according to claim 22, wherein said platform is pivotal about an axle situated in a central region of the platform, said second connecting element is pivotally connected to said platform through an anterior pivotal mount disposed anterior to said pivotal axle, said first connecting element is pivotally connected to said platform by means of a posterior pivotal mount disposed posterior to the pivotal axle.

24. A prosthetic knee, comprising:
a hydraulic cylinder having a hydraulic piston movably disposed thereinside, so as to form a hydraulic chamber between an interior of said hydraulic cylinder and piston, a first connecting element extending outwardly from said hydraulic cylinder; and
a gas cylinder independent from said hydraulic cylinder said gas cylinder having a gas piston movably disposed thereinside, so as to form a gas chamber between the interior of said gas cylinder and piston, a second connecting element extending outwardly from said gas piston;
whereby motion of said hydraulic piston in one direction causes motion of said gas piston in the opposite direction.

25. The prosthetic knee according to claim 24, further comprising a gas valve arrangement associated with said gas chamber, so that during movement of said hydraulic piston toward a bottom of said hydraulic cylinder said gas chamber is extended, so as to allow a gas to enter the gas chamber via said gas valve arrangement.

26. The prosthetic knee according to claim 25, wherein upon motion of said hydraulic piston away from said bottom of the hydraulic cylinders the respective motion of said gas piston diminishes said gas chamber, so as to compress and discharge said gas from said gas chamber via said valve arrangement.

27. The prosthetic knee according to claim 24, wherein said motion of said hydraulic piston away from said bottom of the hydraulic cylinder is caused by a pressurized hydraulic fluid situated within said hydraulic operational chamber.
